# EUROPEAN PATENT APPLICATION

(11) **EP 4 005 580 A1**
(43) Date of publication of application: **01.06.2022**
(21) Application number: 21210134.9
(22) Date of filing: 24.11.2021
(51) Int. Cl.: A61K 36/07, A61K 36/074, A61K 38/48, A61P 29/00

(54) **NATTOKINASE FOR USE IN THE TREATMENT OF DEEP ENDOMETRIOSIS**

(30) Priority: 26.11.2020 IT 202000028535
(71) Applicant: Napoletano, Ilaria, 04100 Latina (LT) (IT)
(72) Inventor: Napoletano, Ilaria, 04100 Latina (LT) (IT)
(74) Representative: Perani & Partners S.p.A.

(57) **Abstract**

The object of the invention relates to the use of nattokinase in the treatment of deep endometriosis, preferably in association with Shiitake extract and still more preferably in association with Shiitake extract and Reishi extract.

## Description

### FIELD OF THE INVENTION

The field of the present invention relates to the use of nattokinase in the treatment of deep endometriosis, in particular administered orally in association with Shiitake extract and still better in association with Shiitake extract and Reishi extract.

### BACKGROUND ART

Endometriosis is a disease characterized by the presence of endometrium, mucosa which normally covers exclusively the uterine cavity, outside the uterus and can affect women already with their first menstruation (menarche) and accompany them until menopause.

In Italy, 10-15% of women of reproductive age suffer from endometriosis; the disease affects about 30-50% of women who are infertile or have difficulty conceiving. There are at least 3 million women with an established diagnosis. The peak occurs between the ages of 25 and 35, but the disease can also appear in lower age groups.

The diagnosis often comes after a long and expensive path, most often experienced with serious psychological repercussions for the woman.

Regarding the causes, one of the accredited hypotheses is the passage, caused by uterine contractions which occur during menstruation, of fragments of the endometrium from the uterus into the tubes and from these into the abdomen, with implantation on the peritoneum and on the surface of the pelvic organs, rarely on the liver, diaphragm, pleura and lung.

Endometriosis causes sub-fertility or infertility (30-40% of cases) and the impact of the disease is high and is linked to the reduction of quality of life and direct and indirect costs.

Deep (or infiltrating) endometriosis is the most severe form of this disease: the endometrium penetrates more than five mm into the affected mucous membranes, creating direct and indirect damage to the organs. In this case the most affected areas are the back of the perineum and the pelvic connective tissue or even the pelvic organs.

In deep endometriosis, the symptoms are very varied and include abdominal pain, alterations of intestinal transit (diarrhoea or constipation), intestinal bleeding. The symptoms may worsen in the period before the menstrual cycle.

The diagnosis of deep endometriosis, rather than with a gynaecological examination, which only detects typical alterations of the fornix and/or recto-vaginal septum in some cases, finds valid aid both with transvaginal pelvic ultrasound and through magnetic resonance imaging (MRI) of the pelvis. Sometimes, dual contrast opaque enema, conventional and virtual colonoscopy may also be helpful.

The presence of occult blood in the faeces can also testify to micro-bleeding in the most severe forms of deep endometriosis, whereby the pathological tissue infiltrates the intestinal mucosa and CA125, a typical blood marker, can be used to diagnose this form of endometriosis.

In these cases it is difficult for the doctor to choose the best therapy if the general conditions of the patient, the impact of the quality of life, the history and characteristics of the endometriosis disease, as well as the extension thereof, are not known.

Where surgery is required, the procedure is laparoscopic and very complex, so that it should only be performed in highly specialized centres. In fact, deep endometriosis often requires bowel resection and is burdened by a considerable number of complications, including bladder and rectal dysfunctions resulting from denervation. The simultaneous presence of endometriosis outbreaks in other sites, sometimes with cyst formation, increases the difficulties of these interventions, which sometimes require the passage to the laparotomic path and the assistance of a general surgeon.

Drug therapy is often able to reduce painful symptoms and the size of lesions. Sometimes it is administered to the patient before surgery to induce active lesions and make it more conservative; however, if it is administered after surgery and lasts for long periods of time, it can reduce the number of relapses.

The most commonly used drugs in deep endometriosis are:
- combined oral contraceptives including the most recent extended-regimen contraceptives (SEASONIQUE^{®}) taken in 91-day cycles;
- GnRH agonists (DECAPEPTYL^{®}, which is capable of causing a temporary menopause), which is very effective but aggravated by side effects caused by hypoestrogenism;
- low-dose progestins administered continuously (Norethisterone acetate, 5 mg for 7 days, or Dienogest for the antiproliferative action on both the ectopic and endouterine endometrium,
- Danazole has also been used in direct intravaginal administration, but should be avoided for long periods of time for the androgenic side effects (F. Di Prospero "Endometriosi profonda infiltrante"26.12.2018 (https://fertihelp.it/endometriosi/endometriosi-profonda-infiltrante/);
- aromatase inhibitors known to induce an increased risk of osteoporosis;
- steroidal and non-steroidal anti-inflammatory drugs (or NSAIDs).

Therefore, conventional drug therapies, especially when performed for long periods, can result in a series of side effects inducing women to use them for short periods or not to use them at all.

The Applicant has filed a patent with Publication No. 102016000029331 disclosing the use of Shiitake extract, still better if associated with Reishi extract, for the treatment and prevention of endometriosis.

Nattokinase is an enzyme extracted and purified from a traditional Japanese food called natto̅. Natto̅ is commonly obtained by fermentation of boiled soybeans with the addition of the bacterium *Bacillus natto,* which produces the enzyme. It seems that the consumption of natto̅ significantly contributes to the longevity of the Japanese population. Natto̅ is the only preparation which contains the specific enzyme nattokinase.

Although the name may be misleading, nattokinase is not a kinase enzyme, but a serine protease of the subtilisin family. Its name instead derives from the fact that it is an enzyme produced by natto̅kin, the Japanese name of *Bacillus subtilis var natto.*

Structurally, nattokinase consists of the residue of 275 amino acids, has a molecular weight of 275 Daltons and is presented as an odourless, milky-white powder.

With respect to other fibrinolytic enzymes, such as tissue plasminogen activator, streptokinase, urokinase, lumbrokinase, nattokinase has several advantages: greater activity, no evidence of collateral effect(s), prolonged life-time, low cost and possibility of oral administration (Journal of Water Process Engineering, 2020, 38, 101533*).* In fact, although it should be expected that, like other proteins, said enzyme will be digested and inactivated in the human intestine, some researchers report that nattokinase is active when taken orally (Applied Microbiology and Biotechnology, 2014, 98, 9199-9206*).*

From a pharmacological point of view, when in contact with human blood or blood clots, nattokinase exhibits strong fibrinolytic activity and works by inactivating the plasminogen activator inhibitor 1 (PAI-1). In addition to the fibrinolytic effect, nattokinase seems to have anti-platelet, anti-coagulant, anti-atherosclerotic and lipid reduction properties which all contribute to its known and main use as a modulator of cardiovascular activity (H. Chen, Biomark Insights, 2018, 13, doi: No. 10.1177/1177271918785130).

### SUMMARY OF THE INVENTION

The Applicant has surprisingly found that nattokinase is effective in the treatment of deep endometriosis preferably when administered orally in formulations containing it as an active ingredient in combination with suitable excipients and/or diluents for oral formulation.

It is thereby advantageously possible to specifically treat *deep* endometriosis with an active ingredient or principle, namely nattokinase, which is effective, even when administered orally, at low cost and without any evidence of long-term side effects unlike in conventional drug therapies.

### DETAILED DESCRIPTION OF THE INVENTION

For the purposes of the present invention, the definition "containing" or "comprising" does not exclude the possibility of additional components being contained in addition to those listed after said definition, while the definition "consisting of" excludes such an eventuality.

The nattokinase for use according to the present invention is preferably administered in oral formulations containing it as an active agent in combination with suitable excipients and/or diluents.

Preferably excipients and/or diluents adapted for oral formulation(s) means chemicals known to those skilled in the art which, for example, play a role as diluents, binders, disintegrants, glidants, lubricants, anti-adherents, stabilizers, colourants, sweeteners, flavourings, absorbents and other.

Preferably, said oral formulations, which contain nattokinase as an active ingredient, are formulations of a conventional type and known to those skilled in the art, such as tablets, capsules or powders dispersible in water.

The nattokinase for use according to the present invention is preferably administered orally at dosages comprised between 2000 FU and 5000 FU and preferably from once to twice daily.

More preferably, the nattokinase for use according to the present invention is administered orally at doses of 2000 FU once daily.

In particular, the activity of the nattokinase is commonly measured with the number of "fibrinolytic units" (FU). This represents the official measurement of nattokinase activity also adopted by the *Japan Health Food Authorization.* The FU unit is defined according to a specific test of the ability of a preparation to dissolve fibrin, i.e., the protein found in clots (Astrup, T.; Mullertz, S. The fibrin plate method for estimating fibrinolytic activity. Arch. Biochem. Biophys. 1952, 40, 346-351*).*

Preferably the nattokinase for use according to the present invention is administered orally in combination with an oral administration of a Shiitake extract possibly also in combination with a Reishi extract.

Even more preferably, the oral administration of the nattokinase for use according to the present invention is administered together with both extracts, thus together with said Shiitake extract and said Reishi extract.

Preferably the Shiitake and Reishi extracts are themselves contained in the same oral formulation as that containing nattokinase or in one or two oral formulations different from that comprising nattokinase, more preferably they are contained in a single oral formulation distinct from that containing nattokinase.

In the oral formulation(s) the Shiitake extract is preferably administered at daily dosages comprised between 100 mg and 1000 mg, preferably between 500 mg and 800 mg.

In the oral formulation(s) Reishi extract is preferably administered at daily dosages comprised between 100 mg and 1000 mg, preferably between 500 mg and 800 mg.

Preferably said oral formulation(s) are food supplements.

For the purposes of the present invention, food supplement means a formulation which falls under the definition of Directive 2002/46/EC and subsequent amendments. In this legislation, food supplements are precisely defined as: "foodstuffs intended to supplement the common diet and constituting a concentrated source of nutrients, such as vitamins and minerals, or of other substances having a nutritional or physiological effect, in particular, but not exclusively, amino acids, essential fatty acids, fibres and extracts of plant origin, both mono- and multi-compound, in predominant forms".

## Claims

1. Nattokinase for use in the treatment of deep endometriosis.

2. Nattokinase for use according to claim 1, wherein the nattokinase is administered in oral formulations containing it as an active agent in combination with suitable excipients and/or diluents.

3. Nattokinase for use according to claim 2, wherein the nattokinase is administered orally at dosages comprised between 2000 FU and 5000 FU from once to twice daily.

4. Nattokinase for use according to claim 3, wherein the nattokinase is administered orally at dosages of 2000 FU once daily.

5. Nattokinase for use according to any one of claims from 2 to 4, wherein the nattokinase is administered orally in association with an oral administration of Shiitake extract possibly in association with a Reishi extract.

6. Nattokinase for use according to claim 5, wherein the oral administration of the Shiitake extract is associated with the oral administration of the Reishi extract.

7. Nattokinase for use according to claim 6, wherein the Shiitake and Reishi extracts are themselves contained in the same oral formulation as that containing nattokinase or in one or two oral formulations different from that comprising nattokinase, preferably they are contained in a single oral formulation distinct from that containing nattokinase.

8. Nattokinase for use according to any one of claims from 5 to 7, wherein the Shiitake extract is administered at daily dosages comprised between 100 mg and 1000 mg, preferably 500 mg.

9. Nattokinase for use according to any one of claims from 5 to 7, wherein the Reishi extract is administered at daily dosages comprised between 100 mg and 1000 mg, preferably between 500 mg and 800 mg.

10. Nattokinase for use according to any one of claims from 7 to 9, wherein said oral formulation(s) are food supplements.
